# EUROPÄISCHE PATENTSCHRIFT

(11) **EP 0 854 708 B1**
(45) Veröffentlichungstag und Bekanntmachung des Hinweises auf die Patenterteilung: **12.12.2001**
(21) Anmeldenummer: 96934651.9
(22) Anmeldetag: 11.10.1996
(51) Int. Cl.: A61K 31/035

(54) **LIPIDALKOHOLE ALS NEUE IMMUNSUPPRESSIVE UND ANTIVIRALE ARNEIMITTEL**
LIPID ALCOHOLS AS NEW IMMUNOSUPPRESSIVE AND ANTIVIRAL DRUGS
ALCOOLS LIPIDIQUES COMME NOUVEAUX MEDICAMENTS IMMUNOSUPPRESSEURS ET ANTIVIRAUX

(30) Priorität: 14.10.1995 DE 19538402
(43) Veröffentlichungstag der Anmeldung: 29.07.1998
(73) Patentinhaber: Heidelberg Pharma Holding GmbH, 69126 Heidelberg (DE)
(72) Erfinder: ZILCH, Harald, D-68305 Mannheim (DE); HERRMANN, Dieter, D-69126 Heidelberg (DE); OPITZ, Hans-Georg, D-69469 Weinheim (DE)
(74) Vertreter: Patentanwälte Zellentin & Partner
(86) Internationale Anmeldenummer: EP9604438
(87) Internationale Veröffentlichungsnummer: WO9714410

(56) Entgegenhaltungen:
- WO-A-91/05558
- DE-A- 3 638 126
- DE-A- 3 929 217

## Beschreibung

Die vorliegende Erfindung betrifft Lipidalkohole als neue immunsuppressive und antivirale Arzneimittel. Lipidalkohole sind als Zwischenprodukte zur Herstellung von z. B. Phosphocholinen oder Liponucleotiden bekannt. Solche Zwischenprodukte sind beispielsweise in folgenden Patentanmeldungen und Literaturstellen beschrieben: [J. Med. Chem. 34 1377 (1991), Tetrahedron Lett. 26, 1167 (1985), Gazz. Chim. Ital. 116, 25 (1986), Lipids 22, 947 (1987), EP 90 11 6298, DE 36 38 126, EP 0 050 327]. In DE 39 29 217.7 und WO 91/05558 werden Lipide mit Heteroatom-unterbrochenen Ketten in Phosphocholinen beschrieben und die Schriften EP 0 350 287, WO 90 /00555, PCT/EP 93/00294, PCT/EP 93/00295, EP 0545966 und PCT/EP 93/02101 zeigen die Verwendung entsprechender Lipidteile als spezifische Carrier in kovalenten Konjugaten mit Nucleosid-Monophosphaten auf.

Eine pharmakologische Wirkung der als Zwischenprodukt bei der Herstellung verwendeten Lipidalkohole ist in keinem Fall beschrieben.

Auch bei einer routinemäßigen Untersuchung dieser Verbindungen auf antivirale Wirkung in den bekannten Testsystemen für HIV (z.B. MT2 / MTT-Test oder entsprechend Tests mit M/M etc.) konnte (bis 100 ug/ml) keine direkte antiretrovirale Wirkung festgestellt werden.

Gegenstand der vorliegenden Erfindung sind neue immunsuppressive and antivirale Arzneimittel unter Verwendung von Lipidalkohplen der allgemeinen Formeln I und II, 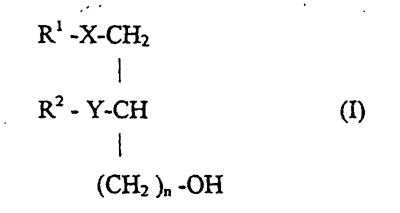 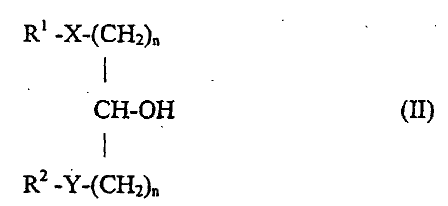 in denen
- R¹: eine geradkettige oder verzweigte, gesättigte oder ungesättigte Alkylkette mit 1-30 Kohlenstoffatomen, die gegebenenfalls ein- oder mehrfach durch Halogen, C₁-C₆-Alkoxy-, C₁-C₆-Alkylmercapto-, C₁-C₆-Alkoxy-carbonyl-, Carboxy, C₁-C₆-Alkylsulfinyl- oder C₁-C₆-Alkylsulfonyl-Gruppen substituiert sein kann,
- R²: Wasserstoff, eine geradkettige oder verzweigte, gesättigte oder ungesättigte Alkylkette mit 1-20 Kohlenstoffatomen, die gegebenenfalls ein- oder mehrfach durch Halogen, C₁-C₆-Alkoxy-, C₁-C₆-Alkylmercapto-, C₁-C₆-Alkoxycarbonyl- oder C₁-C₆-Alkylsulfonylgruppen substituiert sein kann,
- X: einen Valenzstrich, Oxicarbonyl, Carbonyloxy, Amidocarbonyl, Carbonylamido, Sauerstoff, Schwefel, die Sulfinyl- oder die Sulfonylgruppe darstellt,
- Y: einen Valenzstrich, Oxicarbonyl, Carbonyloxy, Amidocarbonyl, Carbonylamido, Sauerstoff, Schwefel, die Sulfinyl- oder die Sulfonylgruppe darstellt,
- n: einen ganzzahligen Wert von 1 bis einschließlich 5 darstellen kann, wobei zumindest einer der Reste R¹ oder R² folgende Bedeutung annimmt, bei R¹ eine geradkettige oder verzweigte, gesättigte Alkylkette mit 7-18 Kohlenstoffatomen, die gegebenenfalls ein- oder mehrfach durch Halogen, C₁-C₆-Alkoxy-C₁-C₆-Alkylmerapto- oder C₁-C₆-Alkylsulfonylgruppen substituiert sein kann, bedeutet
oder bei der R² eine geradkettige oder verzweigte, gesättigte Alkylkette mit 6-16 Kohlenstoffatomen, die noch durch eine C₁-C₆-Alkoxy- oder C₁-C₆-Alkylmercaptogruppe bzw. Halogen substituiert sein kann, bedeutet, sowie deren Tautomere und diese Verbindungen enthaltende Kombinationen mit anderen Wirkstoffen.

Da die Verbindungen der allgemeinen Formeln I und II asymetrische Kohlenstoffatome enthalten, sind auch sämtliche optisch aktiven Formen und racemischen Gemische dieser Verbindungen Gegenstand der vorliegenden Erfindung. Soweit möglich, schließt die Erfindung auch pharmakologisch unbedenkliche (Säureadditions-) Salze mit ein.

Besonders bevorzugt sind fiir R¹ unverzweigte gesättigte Alkylreste mit 8-15 Kohlenstoffatomen. R¹ stellt insbesondere den Nonyl-, Decyl-, Undecyl-, Dodecyl-, Tridecyl- oder Tetradecylrest dar.

Als C₁-C₆-Alkoxy-Substituenten von R¹ kommen vorzugsweise die Methoxy-, Ethoxy-, Butoxy- und die Hexyloxygruppen in Frage. Ist R¹ durch einen C₁-C₆-Alkylmercaptorest substituiert, versteht man darunter insbesondere den Methylmercapto-, Ethyl-mercapto-, Propylmercapto-, Butylmercapto- und den Hexylmercaptorest.

R² ist bevorzugt eine geradkettige C₈-C₁₅-Alkylgruppe, die noch durch eine C₁-C₆-Alkoxygruppe oder eine C₁-C₆-Alkylmercaptogruppe substituiert sein kann. R² stellt insbesondere eine Octyl-, Nonyl-, Decyl, Undecyl-, Dodecyl-, Tridecyl- oder Tetradecylgruppe dar. Als C₁-C₆-Alkoxysubstituenten von R² kommen vorzugsweise die Methoxy-, Ethoxy-, Propoxy-, Butoxy- und die Hexyloxygruppe in Frage. Ist R² durch einen C₁-C₆-Alkylmercaptorest substituiert, versteht man darunter insbesondere den Methylmercapto-, Ethylmercapto-, Butylmercapto- und Hexylmercaptorest.

Bevorzugt sind auch Verbindungen der allgemeinen Formel I, in denen R² das Wasserstoffatom darstellt und Y gleich Sauerstoff oder Valenzstrich ist.

Insbesondere bevorzugt sind dabei Verbindungen, in denen Y gleich Valenzstrich ist, R² für Wasserstoff steht, X die oben angegebene Bedeutung hat und R¹ einen Alkylrest mit 12-25 Kohlenstoffatomen darstellt.

Besonders bevorzugt ist in diesen Kombinationen für R¹ der unverzweigte, gesättigte C₁₂ -C₂₅ -Alkylrest, der über X gleich Schwefel an den Grundkörper gebunden ist.

X bevorzugt gleich Schwefel, Sulfinyl oder Sulfonyl und Y gleich Sauerstoff.

n ist bevorzugt gleich 1 bis 3, besonders bevorzugt jedoch gleich 1.

Die Heteroatome X und Y im Lipidteil können nur in Ausnahmefällen durch die aus Lecithin bekannten Carbonsäureester ersetzt werden, da sonst schon im Serum oder in der Leber (first pass-effect) eine hydrolytische Spaltung zu den entsprechenden Lysolecithin-Derivaten oder Glycerolestern mit entsprechend schnellerer Elimination der pharmakologisch aktiven Substanz erfolgen würde. Die Thioether- bzw. Ether- lipide (X, Y = O,S) dieser Anmeldung zeigen diese Spaltung im Serum verschiedener Spezies, inklusive des Menschen, nicht.

Die Therapie von Malignomen und bösartigen Neoplasien (Karzinome, Sarkome, hämatologische Neoplasien), von entzündlichen Erkrankungen oder von organspezifischen und generalisierten Autoimmunerkrankungen sowie von (Retro-Viren hervorgerufenen Erkrankungen, wie beispielsweise AIDS, ARC (AIDS related complex), Cytomegalie, Herpes oder Hepatitis, ist neben der unzureichenden Wirksamkeit der eingesetzten therapeutischen Wirkstoffe häufig mit extremen Nebenwirkungen verbunden. Dieser Effekt ist mit der geringen Invivo-Selektivität bzw. der eingeschränkten therapeutischen Breite der eingesetzten pharmakologisch aktiven Substanzen zu erklären. Die günstigen pharmakologischen In-vitro-Eigenschaften der jeweiligen Verbindungen sind oft nicht auf die In-vivo-Verhältnisse übertragbar. Seit Jahren versucht man deshalb durch Modifizierung der chemischen Struktur von pharmakologisch aktiven Substanzen neue Substanzen zur Verfügung zu stellen, die verbesserte Eigenschaften hinsichtlich der therapeutischen Breite aufweisen. Ferner werden oft neue pharmazeutische Darreichungsformen mit diesem Ziel entwickelt. Dabei soll insbesondere die unerwünschte Wechselwirkung mit gesunden Zellen/Geweben vermieden werden.

Die erfindungsgemäßen Verbindungen haben In-vitro und In-vivo keinerlei toxische Effekte gezeigt, bis zur höchsten geprüften Konzentration bzw. Dosis von 100 ug/ml bzw. 100 mg/kg.

Die Verbindungen der Formeln I und II sind geeignet zur Behandlung von (retro)viralen Infektion beim Menschen, wie der anhaltenden generalisierten Lymphadenopathie (PGL), dem fortgeschrittenen Stadium des AIDS-verwandten Komplex (ARC) und dem klinischen Vollbild von AIDS.

Von therapeutischem Interesse ist die Hemmwirkung auf HI-Viren (HIV 1 und HIV 2), den Verursachern der Immunschwäche-Erkrankung AIDS. Zur Behandlung von AIDS ist heute z. B. 3'-Azido-3'-desoxythymidin (DE-A-3608606) zugelassen. Jedoch limitieren toxische Nebenwirkungen des 3'-Azido-3'-desoxythymidins, z. B. auf das Knochenmark den Einsatz dieses Medikamentes. Die Verbindungen der allgemeinen Formeln I und II besitzen diese Nachteile nicht. Sie wirken antiviral/antiretroviral, ohne in pharmakologisch relevanten Dosen (cyto)toxisch zu sein.

Die Verbindungen der vorliegenden Erfindung und ihre pharmakologischen Zubereitungen können in Kombination mit anderen Arzneimitteln zur Behandlung und Prophylaxe der oben genannten Infektionen eingesetzt werden. Beispiele für weitere Arzneimittel beinhalten Mittel, die zur Behandlung und Prophylaxe von HIV-Infektionen oder diese Krankheit begleitende Erkrankungen einsetzbar sind wie 3'-Azido-3'-desoxythymidin, 2',3'-Didesoxynucleoside wie z. B. 2',3'-Didesoxyinosin, acyclische Nucleoside (z. B. Acyclovir) oder nicht-nucleosidische RT-Inhibitoren, wie z. B. HEPT, Nevirapin oder L-697,661 und entsprechende Derivate. Die Verbindungen der vorliegenden Erfindung und die anderen Arzneimittel können jeweils einzeln, gleichzeitig gegebenenfalls in einer einzigen oder zwei getrennten Formulierungen oder zu unterschiedlichen Zeiten verabreicht werden.

Es wurde ferner überraschenderweise gefunden, daß auch Lipidalkohole der allgemeinen Formeln I und II eine immunsuppressive oder anti(retro)-virale Wirkung zeigen. Insbesondere eignen sie sich zur Therapie und Prophylaxe von Infektionen, die durch DNA-Viren, wie z. B. HSV-1, HSV-2, CMV, VZV, EBV, HBV, etc. oder RNA-Viren, wie z. B. HTLV-I und -II, HIV-1 und -2, Visna, andere Onko-Viren, etc., verursacht wurden.

Die Verbindungen der allgemeinen Formeln I und II haben überraschenderweise eine antivirale/antiretrovirale In-vitro-Aktivität gezeigt, die zwar nicht direkt die Vermehrung von DNA- bzw. RNA-Viren hemmt, jedoch die Infektiösität der gebildeten Viruspartikel beeinflußt. Die Replikation wird durch die beanspruchten Verbindungen durch Hemmung des Virus-Assembly dahingehend beeinflußt, daß fast nur noch nicht-infektiöse Viruspartikel freigesetzt werden.

Diese Wirkung konnte z. B. für Retroviren auch In-vivo im Friend Virus Leukemie (FVL)-Modell an der Maus nachgewiesen werden.

Außerdem konnte im FVL-Modell ein synergistischer Effekt mit AZT u.a. Reverse Transkriptase (RT) und Non-RT Inhibitoren gezeigt werden. Die gleichzeitige Behandlung mit nicht-wirksame Einzeldosen des Lipidalkohols und des Nucleosids bzw. RT-Inhibitoren, non RT-Inhibitoren etc., zeigte eindeutig synergistische Effekte auf die Überlebenszeit von FVL-infizierten Mäusen.

Die Lipidalkohole der allgemeinen Formeln I und II zeigen durchweg eine große therapeutische Breite, sie haben eine sehr hohe Verweildauer im Körper, die Bioverfügbarkeit ist sehr gut und die oft als kritischer Faktor bekannte Membrangängigkeit (z. B. Zellmembran, Blut-Hirn-Schranke, etc.) ist überdurchschnittlich gut. Die Verbindungen zeigen nur eine langsame Elimination und besitzen in pharmakologisch/therapeutisch relevanten Dosisbereichen keine Knochenmark- oder andere, limitierende Organtoxizitäten.

Insbesondere eignen sich die beanspruchten Verbindungen für die Therapie der angegebenen Erkrankungen in Kombination mit anderen Arzneimitteln/Wirkstoffen.

Die zur Kombination eingesetzte pharmakologisch aktive Substanz kann beispielsweise zytostatische, zytotoxische, antitumorale, antivirale, antiretrovirale, immunsupprimierende oder immunstimulierende Wirkung aufiveisen.

Als pharmakologisch aktive Subtanz kommt eine solche Verbindung in Frage, die z.B. das Tumorwachstum verlangsamt; eine in die DNA und/oder RNA interkalierende Substanz ist, die Topoisomerase I und II hemmt, ein Tubulinhemmer ist, ein Alkylanz ist, eine die Ribosomen inaktivierende Verbindung ist, ein Tyrosinphosphokinase-Inhibitor ist, ein Differenzierungsinduktor ist, ein Hormon, Hormonagonist oder Hormonantagonist ist, eine Substanz ist, welche die pleiotrope Resistenz gegenüber Zytostatika verändert, ein Calmodulin-Inhibitor ist, ein Proteinkinase C-Inhibitor ist, ein P-Glucoprotein-Inhibitor ist, ein Modulator der mitochondrial gebundenen Hexokinase ist, ein Inhibitor der γ-Glutamylcysteinsynthetase oder der Gluthathion-S-Transferase ist, ein Inhibitor der Superoxiddismutase ist, ein Inhibitor der Reversen Transkriptase von HIV-1 und-2 ist.

Die pharmakologisch aktive Substanz kann ferner eine antiinflammatorische, antirheumatische, antiphlogistische, analgetische oder antipyretrische Wirkung äufweisen. Sie kann ferner ein Antiarrhytmikum Calciumantagonist, Antinistaminikum, ein Hemmer der Phosphodiesterase oder ein Sympathomimetikum/-lytikum bzw. Parasympathomimetikum / -lytikum sein. Außerdem sind solche Substanzen geeignet, die gezielt mit dem Zellkern der Zielzellen in Wechselwirkung treten und auf der Ebene der DNA oder RNA in das molekulare Geschehen eingreifen, wie z. B. (Anti)sense-Oligonukleotide, DNA-Fragmente, und die für die Gentherapie verwendet werden können.

Pharmakologisch aktive Substanzen für die Kombination sind beispielsweise:
AZT (Azidothymidin), FLT (Fluorthymidin), 5-FU (5-Fluoruridin), 6-MPR, Fludarabin, Cladribin, Pentostatin, ara-C, ara-A, ara-G, ara-H, Acyclovir, Ganciclovir, Doxorubicin, 4'-epi-Doxorubicin, 4'-Desoxy-doxorubicin, Etoposid, Daunomycin, Idarubicin, Epirubicin, Mitoxantron, Vincristin, Vinblastin, Taxol, Colchicin, Melphalan, 3'-Desoxy- 2-fluoradenosin, FdA, 5-Ethinyluracil-9-β-D-arabinofuranosid, 5-Propinyluracil-9-ß-D-arabinofuranosid, d4T, ddU, ddI, ddA, d2T, 2'-Desoxy-2',2'-difluorcytidin, 5-Trifluor-methyl-2'-desoxyuridin, 5-Chlor-2',3'-didesoxy-3'-fluororidin, 3'-Desoxy-3'-fluor-myoinositol, Neplanocin A, Ribavirin, Myoinositol, Fialuridin, 3TC, Lamivudin, Doxifluridin, Tegafur, Hypericin, Pseudohypericin, Usevir, Famciclovir, Penciclovir, Carvedilol, Actinomycin A, Bleomycin, Daunorubicin, Floxuridin, Mithramycin, Mitomycin C, Mitoxanthron, Streptozotocin, Vindesin, Netilmycin, Amikacin, Gentamycin, Streptomycin, Kanamycin A, Tobramycin, Neomycin B, Plicamycin, Amphotericin B, Vancomycin, Foscamet, Idoxuridin, Trifluridin, Vidarabin sowie Morphine, Prostaglandine, Leukotriene oder Cyclosporine. Ferner kommen in Frage: Terfenadin, Dexamethason; Terbutalin; Prednisolon; Fenoterol; Orciprenalin; Salbutamol; Isoprenalin; Muscarin; Bupranolol; Oxyphenbutazon; Östrogen; Salicylsäure; Propanolol; Ascorbinsäure; Spongiadiol; Diclofenac, Isospongiadiol; Flufenaminsäure; Digoxin; 4-Methylaminophenazon; Allopurinol; Theophyllin; Epoprostenol; Nifedipin; Chinin; Reserpin; Methotrexat; Chlorambucil; Spergualin; Ibuprofen; Indomethacin; Sulfasalazin; Penicillanamin; Chioroquin; Thalidomid.

Bevorzugte pharmakologisch aktive Substanzen sind auch beispielsweise Peptide, Proteine und Oligonucleotide, wie z. B. Corticotropin, Calcitonin, Desmopressin, Gonadotropin, Goserelin, Insulin, Zypressin, beta-Melanotropin, alpha-Melantropin, Muramyldipeptid, Oxytocin, Vaopressin, FK-506, Cyclosporine, Octreotid oder Enalkiren.

Die oben erwähnten pharmakologisch aktiven Substanzen und die daraus herzustellenden Kombinationen stellen neue Beispiele dar und schränken den erfindungsgemäßen Gedanken nicht ein.

Die Verbindungen der vorliegenden Erfindung eignen sich auch zur Therapie und Prophylaxe von Malignomen, Neoplasmien, Karzinomen, Sarkomen oder Leukämien.

Die Verbindungen zeigen außerdem eine immunsuppressive Wirkung und können daher zur Therapie von organspezifischen oder generalisierten Autoimmunerkrankungen, wie beispielsweise rheumatoide Arthritis, systemischer Lupus erythematosus, chronic graft-vs.-host-disease, Multiple Sklerose, etc., oder zur Verhinderung der allogenen oder semiallogenen Transplantatabstossung, wie z. B. von Niere, Leber, Lunge, Herz etc. eingesetzt werden.

Im Vergleich zu bisher zur Behandlung von maligenen Tumoren eingesetzten Verbindungen besitzen die erfindungsgemäßen Verbindungen neben einer guten Wirksamkeit eine bedeutend geringere Toxizität und damit eine größere therapeutische Breite. Sie besitzen dadurch den Vorteil, daß die Verabreichung dieser Verbindungen in Form ihrer Arzneimittelformulierung über einen längeren Zeitraum hinweg kontinuierlich durch geführt werden kann und die Absetzung des Präparates oder eine intermittierende Verabreichung, die bei den heute in der Tumortherapie eingesetzten Cytostatika häufig üblich bzw. aufgrund deren unerwünschten Nebenwirkungen unumgänglich ist, vermieden werden kann.

Die erfindungsgemäßen Verbindungen wirken immunsuppressiv oder antitumoral, ohne in pharmakologisch relevanten Dosen unspezifisch (cyto)toxisch zu sein.

Auch in Kombination mit anderen antiviralen/antiretroviralen, immunsuppressiven oder antitumoralen Arzneimitteln/Wirkstoffen zeigen sich Vorteile der erfindungsgemäßen Verbindungen.

Aufgrund eines synergistischen Effekts der Lipidalkohole der Formel I oder II mit z. B. Nucleosiden kann der Dosisanteil dieser, in höheren Dosen meist toxischen Verbindungen gesenkt werden and damit unerwünschte Nebenwirkungen gemildert oder teilweise vollständig beseitigt werden.

Die Kombination kann grundsätzlich eine alternierende oder eine gleichzeitige Gabe von Lipidalkohol und des zu kombinierenden Wirkstoffes sein.
Der Lipidalkohol kann auch in Form eines Prodrug vorliegen, d.h. er wird erst in vivo metabolisch in Verbindungen der Formel I und II umgewandelt. Solche Prodrugs der Formel I sind bevorzugt solche bei denen die Hydroxygruppe des Glycerols einen Phosphatester oder Pyrophosphatesters bildet. Die Phosphatgruppe oder Pyrophosphatgruppe kann nochmals verestert sein.

Bei vielen Erkrankungen, wie z. B. AIDS, Tumorerkrankungen, etc. kann die Kombination eines Lipidalkohols der Formeln I und II mit mehr als einem zusätzlichen Arzneimittel/Wirkstoff ferner dann von Vorteil sein, wenn z. B. Resistenzentwicklung eintritt.

Für die Behandlung von AIDS-Patienten z.B. ist eine Kombination aus
a) Lipidalkohol der Formel I oder II
b) Nucleosid und
c) nichtnucleodischem RT-Inhibitor, Protease-Inhibitor oder tat-Inbibitor von therapeutischem Vorteil gegenüber der jeweiligen Monotherapie.

Durch entsprechende Vergleichsversuche konnte gezeigt werden, daß die therapeutischen Effekte der bekannten Diacylglycerole in vivo den Thioether- oder Etherlipidalkoholen dieser Anmeldung unterlegen sind. Dies ist auf eine unspezifische Hydrolyse der Fettsäureester zurückzuführen. Die nicht hydrolysierbaren Thioether- und Etherreste zeigen demgegenüber metabolische Stabilität.

Der Lipidalkohol mit seiner lecithinartigen Struktur, die für den beanspruchten Effekt essentiell ist, zeigt bei optimaler Kettenlänge eine gute Membrangängigkeit (gute Überwindung der Resorptionsbarrieren) und einen Depoteffekt.
Durch hohe Bindung der Verbindungen gemäß Formel I und II an Plasma- und Gewebeproteine wird ferner die Verteilung in vivo verbessert. Durch normale Biotransformation wird der Lipidalkohol primär vom Thioester (X=S) zum Sulfoxid (X=SO) oxidiert, was aber aufgrund der equipotenten Wirkung des Sulfoxids im Vergleich zum Thioester keinen therapeutischen Nachteil darstellt. Durch eine langsame Freisetzung aus intrazellulären oder Membrankompartimenten wird ein niedriger, aber über einen langen Zeitraum konstanter Substanzspiegel gewährleistet, somit die Wirkung verbessert oder toxische Nebeneffekte vermieden.

Die Verbindungen der Formeln I und II können als Wirkstoffe zur Herstellung von Arzneimitteln verwendet werden oder die vorhandene Wirkung einer der erfindungsgemäßen Verbindungen wird durch Kombination mit anderen Arzneimitteln zur Behandlung und Prophylaxe von verschiedenen Erkrankungen noch gesteigert.

Verbindungen der Formel I bzw. II und deren Verwendung als Zwischenprodukte sind in den Anmeldungen WO 92/03462, WO 93/16092, WO 93/16091, WO 94/03465, PCT/EP 94/02123, DE 4402492, DE 4418690, sowie beispielsweise in WO 91/19726; EP 0 350 287; US 5,223,263; US 5,194,654; US 4,921,951; US 4,622,392; US 4,291,024; US 4,283,394 beschrieben.

Die Herstellung der Verbindungen der allgemeinen Formel I und II erfolgte auch analog zu Lipids 22, 947 (1987) und J. Med. Chem. 34, 1377 (1991) sowie der im Stand der Technik zitierten Literatur.

Bewährt hat sich ferner folgende Synthese, ausgehend von Glycerin, die für ein spezielles Beispiel aufgezeigt ist:

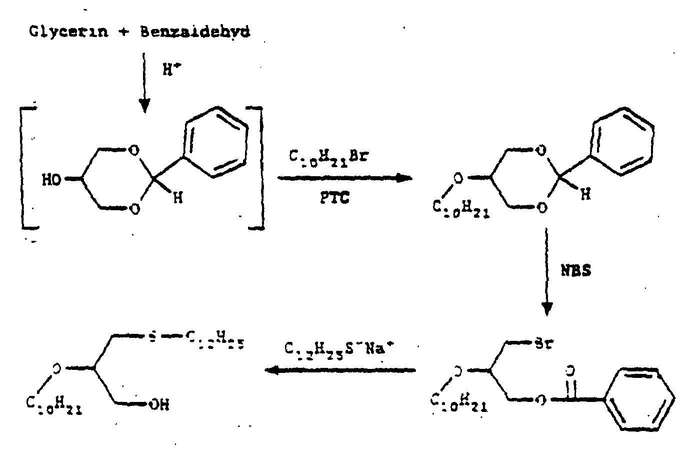

Der so erhaltene Thioether (oder ein analog herzustellendes Derivat mit veränderter Kettenlänge) kann anschließend nach einer dem Fachmann bekannten Methode zum entsprechenden Sulfoxid oder Sulfon oxidiert werden.

Die Arzneimittel enthaltend Verbindungen der Formel I oder II zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z. B. Tartrat- und Zitratpuffer, Ethanol, Komplexbildner, wie Ethylen-diamintetraessigsäure und deren nichttoxische Salze, hochmolekulare Polymere, wie flüssiges Polyethylenoxid zur Viskositätsregulierung.

Flüssige Trägerstoffe für Injektionslösungen müssen steril sein und werden vorzugsweise in Ampullen abgefüllt. Feste Trägerstoffe sind beispielsweise Stärke, Lactose, Mannit; Methylcellulose, Talkum, hochdisperse Kieselsäuren, höher molekulare Fettsäuren wie Stearinsäure, Gelatine, Agar-Agar, Calciumphosphat, Magnesiumstearat, tierische und pflanzliche Fette, feste hochmolekulare Polymere, wie Polyethylenglycole, etc. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 1 - 100 mg, vorzugsweise 2 - 80 mg pro Tag und pro kg Körpergewicht appliziert. Bevorzugt ist es, die Tagesdosis auf 1 - 3 Applikationen zu verteilen, wobei bei jeder Applikation 1 - 2 Tabletten mit einem Wirkstoffgehalt von 5 - 500 mg verabreicht werden. Die Tabletten können auch retardiert sein, wodurch sich die Anzahl der Applikationen pro Tag auf 1 - 2 vermindert. Der Wirkstoffgehalt der retardierten Tabletten kann 2 - 2000 mg betragen. Der Wirkstoff kann auch durch Dauerinfusion gegeben werden, wobei die Mengen von 5 - 3000 mg pro Tag normalerweise ausreichen.
Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage.
2-Butyhnercaptomethyl-1-octadecanol
2-Decyloxy-1-tetradecanol
2-Dodecyloxy-1-tetradecanol
2-Dodecylmercapto-1-tetradecanol
2-Dodecylmercaptomethyl-1-tridecanol
3-Dodecylmercapto-2-decanoyloxy-1-propanol
2,3-Bis-(octadecanoyl)-1-propanol
4-Dodecylmercapto-3-decyloxy-1-butanol
4-Undecylmercapto-3-undecyloxy-1-butanol
5-Dodecyhnercapto-4-decyloxy-1-pentanol
1,3-Bis-(dodecylmercapto)-2-propanol
3-Dodecanoylamino-2-undecyloxy-1-propanol
1-Dodecylmercapto-3-decyloxy-2-propanol
3-Dodecanoylamino-2-decyloxy-1-propanol

(R)-3-Dodecylmercapto-2-decyloxy-1-propanol
(S)-3-Dodecylmercapto-2-decyloxy-1-propanol

### Beispiel 1

### 5-Decyloxy-2-phenyl-1,3-dioxan

92 g Glycerin wurden mit 106 g (101 ml) Benzaldehyd unter Katalyse von 1 ml Methansulfonsäure in 330 ml Toluol 2,5 Stunden am Wasserabscheider unter Rückfluß erhitzt (18 ml Wasser abgeschieden).

Die klare Lösung wurde auf RT abgekühlt, mit 500 ml Isohexan versetzt und 2 - 3 Stunden im Eisbad gerührt (dicker, weißer Niederschlag, evtl. mehr Isohexan erforderlich).

Nach Zugabe von 1 L conc NaOH und 670 ml Toluol wurde das Isohexan bis zu einer Übergangstemperatur von 90 °C abdestilliert (ca. 700 ml Destillat). Die zweiphasige Lösung wurde dann mit 332 ml Bromdecan und 11,8 g Aliquat versetzt und weitere 2 Stunden bei 85 - 90 °C Innentemperatur gerührt.

Nach dem Abkühlen wurde die wässrige Phase abgetrennt, die organische Phase 4 x mit je 1 L Wasser extrahiert und das Toluol bis 150 °C Außentemperatur und 1 mbar abdestilliert.

Der Rückstand wurde in 2 L Isohexan gelöst, mit 10 g Brilonit gekohlt, abgesaugt und bei -20 °C über Nacht zur Kristallisation gebracht. Der Niederschlag wurde bei tiefer Temperatur abgesaugt, mit 100 ml kaltem Isohexan gewaschen and bei Raumtemperatur im Vakuum getrocknet.

Ausbeute: 261 g/ 81 % d. Th. GC: 95,21 Fl-%

### Benzoesäure-(3-brom-2-decyloxy-propyl)ester

72,6 g 5-Decyloxy-2-phenyl-1,3-dioxan wurden in 480 ml Isohexan gelöst, mit 1,36 g Calciumoxid sowie 39 g N-Bromsuccinimid versetzt und 4 Stunden bei 40 °C gerührt.

Dann wurde auf 20 °C abgekühlt, mit 10 g Aktivkohle versetzt und nach 10 Minuten Rühren von der Kohle abfiltriert.

Das Filtrat wurde bei < 30 °C im Vakuum eingedampft und der Rückstand ohne weitere Reinigung direkt in die nächste Reaktion eingesetzt.

Rohausbeute: 89 g, GC: 92,6%, Stufe I: 0,87 %

### 3-Dodecylmercapto-2-decyloxy-1-propanol

55 ml 1-Dodecylmercaptan in 64 ml Methanol wurden innerhalb von 5 Minuten mit einer Lösung aus 43 ml 30 %iger Nariummethylat-Lösung in 325 ml Methanol versetzt und 30 Minuten bei ca. 25 °C gerührt. Dann wurden 89 g des Rohproduktes der letzten Reaktion in 140 ml Methanol zugesetzt und die Lösung weitere 15 Stunden bei 20 - 25 °C gerührt.

Anschließend wurden 52 ml 2N Salzsäure zugegeben, 1 Stunde bei 35 - 40 °C nachgerührt und die Lösung auf 20 °C abgekühlt. Nach Zugabe von 28 ml 50 %iger Natronlauge wurde 2 Stunden bei 40 - 45 °C gerührt, das Methanol bei 100 mbar bis max. 55 °C im Vakuum entfernt und nach Verdünnen mit 480 ml Wasser mit 480 ml MTB extrahiert. Die organische Phase wurde abgetrennt, mit gesättigter Natriumchlorid-Lösung und Wasser gewaschen und im Vakuum vom Lösungsmittel befreit. Der Rückstand (90 g roh) wurde an Kieselgel 60 mit Toluol/MTB 20/1 als Eluens chromatographisch gereinigt. Aus den Produkt-enthaltenden Fraktionen wurden nach dem Entfernen des Lösemitteis im Rotationsverdampfer 78,8 g Öl isoliert. GC: 96 %.

### Beispiel 2

### Wirksamkeit und Verfräglichkeit im Friend-Virus-Leukämie-Modelll

Weiblichen Balb/c-Mäusen, 6-8 Wochen alt (Iffa Credo), wurden pro Tier jeweils 0,2 ml eines virushaltigen Milzüberstandes am Tag O i. p. inokuliert. Die Tiere wurden täglich von Tag O (Beginn: 1 Stunde nach Virusinokulation) bis Tag 13 i. p. mit der zu untersuchenden Substanz in Dosen von 6,25 mg, 12,5 mg, 25 mg und 50 mg pro kg therapiert.

Vor Therapiebeginn sowie am Tag 13 wurden die Parameter Körpergewicht und kleines Blutbild (WBC, RBC, Hb, Hkt, Plt) sowie am Tag 14 nach dem Töten der Tiere die individuellen Milzgewichte als Parameter für die Virämie bestimmt.

### Beispiel 3

### Wirksamkeit in der HIV-infizierten Zellkultur

Routinemäßig wurden im MT2-System in Mikrotiterplatten mit mind. 4 Konzentrationen Dreifachbestimmungen weitgehend automatisch (Biomek von Beckmann) durchgeführt (Standardabweichung < 5 %). In Parallelansätzen wurde sowohl die Toxizität (Zellen + Substanz) als auch die antivirale Wirkung (Zellen + Substanz + Virus) bestimmt.

MT2-Zellen wurden mit der zu untersuchenden Substanz vorinkubiert und mit HIV-1 (HTLV-III-B, MOI 0,03) infiziert. Der Überstand wurde abgenommen, durch Medium (inkl. Substanz) ersetzt und 7 Tage inkubiert.

Danach erfolgte eine Auswertung nach zytopatischem Effekt (Syncytien), MTT-Test (Vitalität der Zellen) und Überführung des Überstandes zur Neuinfektion.

### Beispiel 4

*Analog zu Beispiel 1 wurden folgende Verbindungen hergestellt:*
1. 3-Undecylmercapto-2-decyloxy-1-propanol
2. 3-Tridecylmercapto-2-decyloxy-1-propanol
3. 3-Undecylmercapto-2-undecyloxy-1-propanol
4. 3-Decylmercapto-2-dodecyloxy-1-propanol
5. 3-Undecylmercapto-2-dodecyloxy-1-propanol
6. 3-Dodecylmercapto-2-dodecyloxy-1-propanol
7. 3-Dodecylmercapto-2-undecyloxy-1-propanol
8. 3-Dodecylmercapto-2-decylmercapto-1-propanol
9. 2,3-Bis-(undecylmercapto)-1-propanol
10. 2,3-Bis-(undecyloxy)-1-propanol
11. 3-Dodecyloxy-2-decyloxy-1-propanol
12. 3-Tridecyloxy-2-decyloxy-1-propanol
13. 3-Decyloxy-2-dodecyloxy-1-propanol
14. 3-Pentadecylmercapto-2-decyloxy-1-propanol
15. 3-Octylmercapto-2-decyloxy-1-propanol
16. 3-Decylmercapto-2-octyloxy-1-propanol
17. 3-Decylmercapto-2-dodecylmercapto-1-propanol
18. 3-Dodecyloxy-2-decylmercapto-1-propanol
19. 3-Decyloxy-2-dodecylmercapto-1-propanol
20. 3-Dodecylmercapto-2-octyloxy-1-propanol
21. 3-Decylmercapto-2-decyloxy-1-propanol
22. 3-Tetradecylmercapto-2-decyloxy-1-propanol
23. 2,3-Bis-(octylmercapto)-1-propanol
24. 3-Hexadecylmercapto-2-decyloxy-1-propanol
25. 3-Decylmercapto-2-hexadecyloxy-1-propanol
26. 3-Hexadecylmercapto-2-hexadecyloxy-1-propanol
27. 2,3-Bis-(decyloxy)-1-propanol
28. 3-Hexadecylmercapto-2-cyclohexyloxy-1-propanol
29. 3-(9-Phenyl-nonylmercapto)-2-decyloxy-1-propanol
30. 3-Dodecylmercapto-2-(9-phenyl-nonyloxy)-1-propanol
31. 3-(2-Methyl-undecyl)mercapto-2-decyloxy-1-propanol
32. 3-(2-Butyl-octyl)mercapto-2-decyloxy-1-propanol

### Beispiel 5

### 3-Dodecylsulfinyl-2-decyloxy-1-propanol

10g 3-Dodecylmercapto-2-decyloxy-1-propanol wurden in 100ml Eisessig gelöst und nach Zugabe von 10ml 30% Wasserstoffperoxid 4h bei Raumtemperatur gerührt. Dann wurde das Lösungsmittel im Rotationsverdampfer entfernt und der Rückstand an Kieselgel 60 mit Ether/Isohexan 1:2 als Eluens chromatographisch gereinigt. Die Produkt-enthaltenden Fraktionen wurden eingedampft und lieferten 7,4g des gewünschten Sulfoxids als Öl.

### Beispiel 6

*Analog zu Beispiel 5 wurden folgende Verbindungen hergestellt:*
1. 3-Undecylsulfinyl-2-decyloxy-1-propanol
2. 3-Tridecylsulfinyl-2-decyloxy-1-propanol
3. 3-Undecylsulfinyl-2-undecyloxy-1-propanol
4. 3-Decylsulfinyl-2-dodecyloxy-1-propanol
5. 3-Undecylsulfinyl-2-dodecyloxy-1-propanol
6. 3-Dodecylsulfinyl-2-dodecylbxy-1-propanol
7. 3-Dodecylsulfinyl-2-undecyloxy-1-propanol

### Beispiel 7

### 3-Dodecylsulfonyl-2-decyloxy-1-propanol

10g 3-Dodecylmercapto-2-decyloxy-1-propanol wurden in 100ml Eisessig gelöst und nach Zugabe von 25ml 30% Wasserstoffperoxid 6h bei 50 Grad gerührt. Danach wurden nochmals 13ml Wasserstoffperoxid zugegeben und weitere 7h gerührt. Dann wurde das Lösungsmittel im Rotationsverdampfer entfernt und der Rückstand an Kieselgel 60 mit Ether/Isohexan 1:1,5 als Eluens chromatographisch gereinigt. Die Produkt-enthaltenden Frak-tionen wurden eingedampft und lieferten 8g des gewünschten Sulfons als Öl.

### Beispiel 8

*Analog zu Beispiel 7 wurden folgende Verbindungen hergestellt:*
1. 3-Undecylsulfonyl-2-decyloxy-1-propanol
2. 3-Tridecylsulfonyl-2-decyloxy-1-propanol
3. 3-Undecylsulfonyl-2-undecyloxy-1-propanol
4. 3-Decylsulfonyl-2-dodecyloxy-1-propanol
5. 3-Undecylsulfonyl-2-dodecyloxy-1-propanol
6. 3-Dodecylsulfonyl-2-dodecyloxy-1-propanol
7. 3-Dodecylsulfonyl-2-undecyloxy-1-propanol

### Beispiel 9

### 1,3-Bis-(dodecylmercapto)-2-propanol

In eine Ethylat-Lösung aus 2,6g Natrium in 100ml Ethanol wurden 26,6ml Dodecanthiol eingetragen und die Lösung 1h bei Raumtemperatur gerührt. Dann wurden 8,5 ml Epibromhydrin innerhalb von 30min zugetropft und über Nacht gerührt. Nach Entfernen des Lösemittels wurde der Rückstand in Ether aufgenommen, zweimal mit Wasser gewaschen und getrocknet. Beim Einengen der Lösung kristallisierte die gerwünschte Verbindung aus. Ausbeute 28,5g (62%).

### Beispiel 10

### 1-Dodecylmercapto-3-decyloxy-2-propanol

Eine Mischung aus 9,52ml 1-Decanol, 4,23ml Epibromhydrin und 3,4g Tetrabutylammonium-hydrogensulfat in 150ml Dichlormethan und 150ml 50% Natronlauge wurde 3h bei Raumtemperatur gerührt. Dann wurde die organische Phase abgetrennt, zweimal mit Wasser gewaschen und eingedampft. Der Rückstand wurde an Kieselgel 60 mit ether/Isohexan 1:15 als Eluens chromatographisch gereinigt. Ausbeute 5,4g Öl.

Dieses Öl wurde nach Lösen in 30ml Ethanol mit einer Mecaptid-Lösung versetzt, die vorher durch Umsetzung von 1,94ml 1-Dodecylmercaptan in 20ml Ethanol mit Natriumethylat hergestellt wurde. Nach Rühren über Nacht bei Raumtemperatur wurde das Lösemittel abdestilliert, der Rückstand in Dichlormethan aufgenommen, zweimal mit Wasser gewaschen und die organische Phase eingedampft. Der Rückstand wurde durch Chromatographie an Kieselgel 60 mit Ether/Isohexan 1:5 als Eluens gereinigt. Ausbeute 3,9g.

### Beispiel 11

### 2-Decyloxy-1-tetradecanol

Eine Suspension aus 1,2g Natriumhydrid 90% in 21ml DMF wurde innerhalb von 15min mit 8,5ml 1-Decanol in 30ml DMF versetzt und 1h nachgerührt.. Dann wurden 15g 2-Brom-tetradecansäuremethylester in 48ml Toluol zugetropft und die Lösung 24h bei Raumtemperatur gerührt. Nach Abdampfen des Lösungsmittels wurde der Rückstand in Ether aufgenommen, mit Wasser gewaschen und die organische Phase eingedampft. Rückstand 18,9g.

Das Öl wurde in 200ml Ether gelöst, mit 1,3g Lithiumaluminiumhydrid versetzt und 1h unter Rückfluß erhitzt. Dann wurde hydrolysien und die Etherphase eingedampft. Der Rückstand (17,4g) wurde durch Säulenchromatographie an Kieselgel 60 mit Ether/Isohexan 1:4 als Eluens gereinigt. Ausbeute 4,26g.

### Beispiel 12

2-Dodecyloxy-1-tetradecanol wurde analog zu Beispiel 11 durch Umsetzung mit Dodecanol erhalten

### Beispiel 13

Die Zellzytotoxizität und die Anti-HIV-1-Aktivität in CEM-SS-Zellen ausgewählter Lipidalkohole ist in Tabelle 1 angegeben.

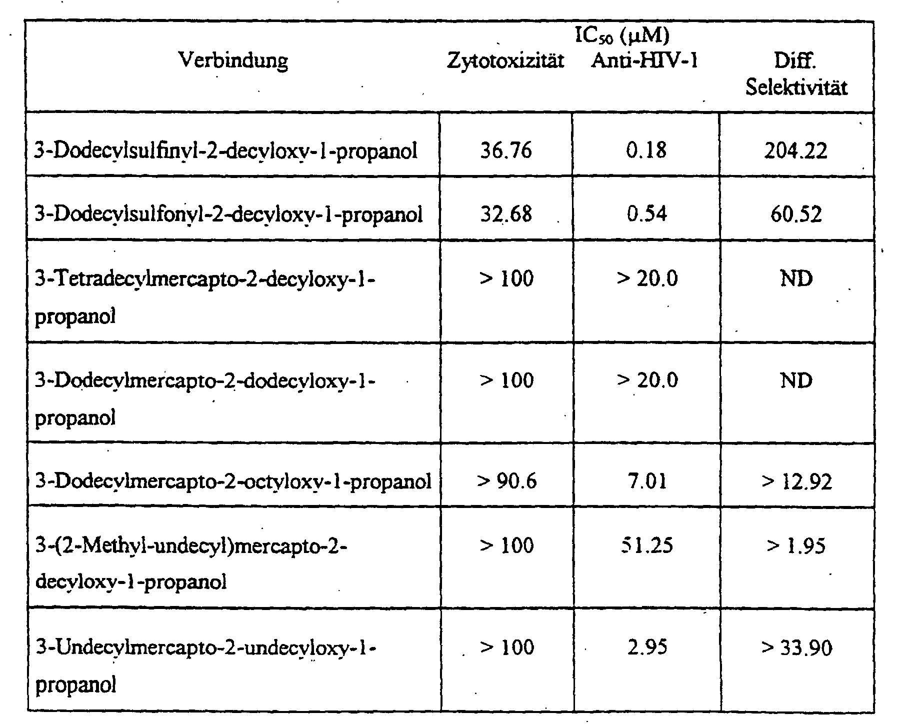

Die Zytotoxizität wurde durch die Aufnahme von TdR-³H in die Gesamt-DNA bei verschiedenen Konzentrationen der angegebenen Verbindungen bestimmt. Die Anti-HIV-1 Aktivität wurde durch einen Standard Plasque assay von CEM-SS-Zell monolayer bestimmt. Die Differentielle Selektivität ergibt sich aus dem Quotienten der Konzentrationenen von IC₅₀ und Anti-HIV-1-Aktivität. N.D. bedeutet nicht bestimmt.

## Patentansprüche

1. Arzneimittel enthaltend ein Lipidalkohol der allgemeinen Formeln I oder II 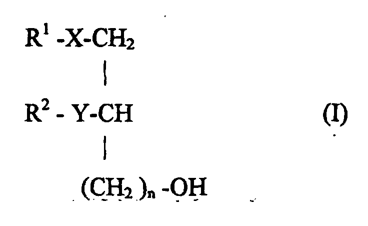 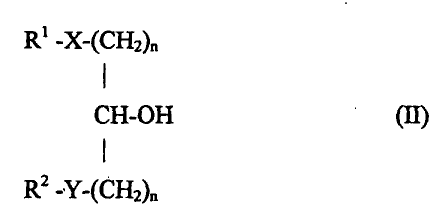 in denen
R¹ eine geradkettige oder verzweigte, gesättigte oder ungesättigte Alkylkette, mit 1-30 Kohlenstoffatomen, die gegebenenfalls ein- oder mehrfach durch Halogen, C₁-C₆-Alkoxy-, C₁-C₆-Alkylmercapto-, C₁-C₆-Alkoxy-carbonyl-, Carboxy, C₁-C₆-Alkylsulfinyl- oder C₁-C₆-Alkylsulfonyl-Gruppen substituiert sein kann,
R² Wasserstoff, eine geradkettige oder verzweigte, gesättigte oder ungesättigte Alkylkette mit 1-20 Kohlenstoffatomen, die gegebenenfalls ein- oder mehrfach durch Halogen, C₁-C₆-Alkoxy-, C₁-C₆-Alkylmercapto-, C₁-C₆-Alkoxycarbonyl- oder C₁-C₆-Alkylsulfonylgruppen substituiert sein kann,
X einen Valenzstrich, Oxicarbonyl, Carbonyloxy, Amidocarbonyl, Carbonylamido, Sauerstoff, Schwefel, die Sulfinyl- oder die Sulfonylgruppe darstellt,
Y einen Valenzstrich, Oxicarbonyl, Carbonyloxy, Amidocarbonyl, Carbonylamido, Sauerstoff, Schwefel, die Sulfinyl- oder die Sulfonylgruppe darstellt,
n einen ganzzahligen Wert von 1 bis 5 einschließlich darstellen kann,
wobei zumindest einer der Reste R¹ oder R² folgende Bedeutung annimmt, bei der R¹ in Formel I oder II eine geradkettige oder verzweigte, gesättigte Alkylkette mit 7-18 Kohlenstoffatomen, die gegebenenfalls ein oder mehrfach durch Halogen, C₁-C₆-Alkoxy, C₁-C₆ Alkyl mercapto, oder C₁-C₆ Alkylsulfonylgruppen substituiert sein kann, bedeutet
oder
bei der R² in Formel I oder II eine geradkettige oder verzweigte, gesättigte Alkylkette mit 6-16 Kohlenstoffatomen, die noch durch eine C₁-C₆-Alkoxy- oder C₁-C₆-Alkylmercaptogruppe oder Halogen substituiert sein kann, bedeutet,
sowie deren Tautomere, Prodrugs und diese Verbindungen enthaltende Kombinationen mit anderen Wirkstoffen, und weitere pharmazeutisch übliche Hilfs- oder Trägerstoffe.

2. Arzneimittel nach Anspruch 1 in Kombination mit anderen Wirkstoffen.

3. Arzneimittel nach einem der Ansprüche 1 oder 2, **dadurch gekennzeichnet, daß** X in Formel I oder II gleich Schwefel, Sulfinyl oder Sulfonyl und Y gleich Sauerstoff ist.

4. Arzneimittel nach einem der Ansprüche 1 bis 3, **dadurch gekennzeichnet, daß** n in Formel I oder II gleich 1 bis 3 ist.

5. Verwendung von Lipidalkoholen der Formel gemäß einem der Ansprüchen 1 bis 4 zur Herstellung von Arzneimitteln zur Behandlung von viralen oder retroviralen Infektionen, Malignomen, Neoplasmien, entzündlichen Erkrankungen oder von Autoimmunerkrankungen.

## Claims

1. Pharmaceutical agent containing a lipid alcohol of the general formulae I and II 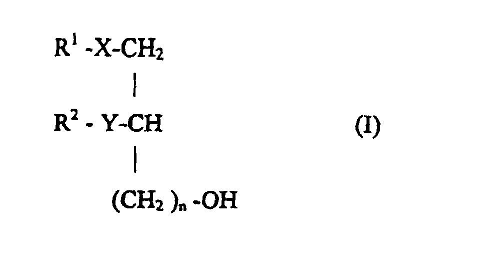 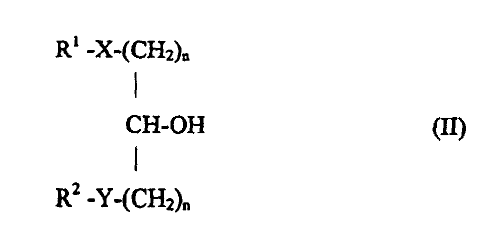 in which
R¹ represents a straight-chained or branched, saturated or unsaturated alkyl chain with 1-30 carbon atoms which can optionally be substituted once or several times by halogen, C₁-C₆ alkoxy, C₁-C₆ alkylmercapto, C₁-C₆ alkoxycarbonyl, carboxy, C₁-C₆ alkylsulfinyl or C₁-C₆ alkylsulfonyl groups,
R² represents hydrogen, a straight-chained or branched, saturated or unsaturated alkyl chain with 1-20 carbon atoms which can optionally be substituted once or several times by halogen, C₁-C₆ alkoxy, C₁-C₆ alkylmercapto, C₁-C₆ alkoxycarbonyl or C₁-C₆ alkylsulfonyl groups,
X represents a valency dash, oxicarbonyl, carbonyloxy, amidocarbonyl, carbonylamido, oxygen, sulphur, a sulfinyl or sulfonyl group
Y represents a valency dash, oxicarbonyl, carbonyloxy, amidocarbonyl, carbonylamido, oxygen, sulphur, a sulfinyl or sulfonyl group
n represents an integer value from 1 to 5 inclusive
wherein at least one of the residues R¹ or R² takes on the following meaning in which R¹ in formula I or II represents a straight-chained or branched, saturated alkyl chain with 7-18 carbon atoms which can optionally be substituted once or several-times by halogen, C₁-C₆ alkoxy, C₁-C₆ alkylmercapto or C₁-C₆ alkylksulfonyl groups
or
in which R² in formula I or II denotes a straight-chained or branched, saturated alkyl chain with 6-16 carbon atoms which can additionally be substitued by a C₁-C₆ alkoxy or C₁-C₆ alkylmercapto group or halogen,
as well as tautomers thereof, prodrugs and combinations containing these compounds with other active substances and further pharmaceutically common auxiliary or carrier substances.

2. Pharmaceutical agent as claimed in claim 1 in combination with other active substances.

3. Pharmaceutical agent as claimed in one of the claims 1 or 2, wherein X in formulae I or II is sulphur, sulfinyl or sulfonyl and Y is oxygen.

4. Pharmaceutical agent as claimed in one of the claims 1 to 3, wherein n in formula I or II equals 1 to 3.

5. Use of lipid alcohols of the formula as claimed in one of the claims 1 to 4 for the production of pharmaceutical agents for the treatment of viral or retroviral infections, malignomas, neoplasias, inflammatory diseases or autoimmune diseases.

## Revendications

1. Médicament contenant un alcool lipidique répondant aux formules générales I ou II 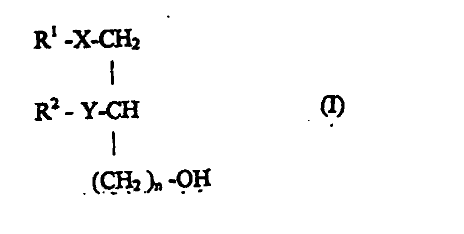 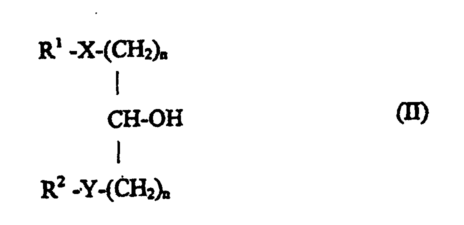 dans lesquelles
R¹ représente un groupe alkyle à chaîne droite ou ramifiée, saturé ou non saturé contenant de 1 à 30 atomes de carbone, qui peut être substitué le cas échéant une ou plusieurs fois par un atome d'halogène, un groupe alcoxy en C₁-C₆, un groupe alkyl(en C₁-C₆)mercapto, un groupe alcoxy(en C₁-C₆)carbonyle, un groupe carboxyle, un groupe alkyl(en C₁-C₆)sulfinyle ou un groupe alkyl(en C₁-C₆)sulfonyle,
R² représente un atome d'hydrogène, un groupe alkyle à chaîne droite ou ramifiée, saturé ou non saturé contenant de 1 à 20 atomes de carbone, qui peut être substitué le cas échéant une ou plusieurs fois par un atome d'halogène, un groupe alcoxy en C₁-C₆, un groupe alkyl(en C₁-C₆)mercapto, un groupe alcoxy(en C₁-C₆)carbonyle ou un groupe alkyl(en C₁-C₆)sulfonyle,
X représente un trait de valence, un groupe oxycarbonyle, un groupe carbonyloxy, un groupe amidocarbonyle, un groupe carbonylamido, un atome d'oxygène, un atome de soufre, un groupe sulfinyle ou un groupe sulfonyle,
Y représente un trait de valence, un groupe oxycarbonyle, un groupe carbonyloxy, un groupe amidocarbonyle, un groupe carbonylamido, un atome d'oxygène, un atome de soufre, un groupe sulfinyle ou un groupe sulfonyle,
n peut représenter un nombre entier de 1 à 5 inclus,
dans lequel au moins un des radicaux R¹ et R² prend la signification indiquée ci-après, dans laquelle R¹, dans la formule I ou II, représente un groupe alkyle saturé à chaîne droite ou ramifiée contenant de 7 à 18 atomes de carbone, qui peut être substitué le cas échéant, une ou plusieurs fois, par un atome d'halogéne, un groupe alcoxy en C₁-C₆, un groupe alkyl(en C₁-C₆)mercapto ou un groupe alkyl(en C₁-C₆)sulfonyle,
ou bien
dans laquelle R², dans la formule I ou II, représente un groupe alkyle saturé à chaîne droite ou ramifiée contenant de 6 à 16 atomes de carbone, qui peut encore être substitué par un groupe alcoxy en C₁-C₆ ou par un groupe alkyl(en C₁-C₆)mercapto,
ainsi que ses tautomères, ses précurseurs et des combinaisons contenant ces composés avec d'autres principes actifs, ainsi que d'autres substances auxiliaires ou de support habituelles dans le domaine pharmaceutique.

2. Médicament selon la revendication 1, en combinaison avec d'autres principes actifs.

3. Médicament selon l'une quelconque des revendications 1 ou 2, **caractérisé en ce que** X, dans la formule I ou II représente un atome de soufre, un groupe sulfinyle ou un groupe sulfonyle et Y représente un atome d'oxygène.

4. Médicament selon l'une quelconque des revendications 1 à 3, **caractérisé en ce que** n, dans la formule I ou II, est égal à 1 - 3.

5. Utilisation d'alcools lipidiques répondant à la formule selon l'une quelconque des revendications 1 à 4, pour la préparation de médicaments destinés au traitement d'infections virales ou rétrovirales, de malignomes, de néoplasies, de maladies inflammatoires ou de maladies autoimmunes.
